# EUROPEAN PATENT SPECIFICATION

(11) **EP 4 178 463 B1**
(45) Date of publication and mention of the grant of the patent: **11.11.2026**
(21) Application number: 20945377.8
(22) Date of filing: 13.07.2020
(51) Int. Cl.: A61B 17/12, A61B 17/00, A61M 25/10

(54) **DEVICES FOR BLOOD VESSEL OCCLUSION**
VORRICHTUNGEN ZUR BLUTGEFÄSSOKKLUSION
DISPOSITIFS POUR L'OCCLUSION D'UN VAISSEAU SANGUIN

(43) Date of publication of application: 17.05.2023
(73) Proprietor: Front Line Medical Technologies Inc., London ON N6H 1L2 (CA)
(72) Inventor: PAREKH, Asha, London, Ontario N6H 1L2 (CA); POWER, Adam, London, Ontario N6H 4P7 (CA)
(74) Representative: WP Thompson
(86) International application number: PCT/CA2020/050976
(87) International publication number: WO 2022/011446

(56) References cited:
- WO-A1-2017/210584
- WO-A1-2020/033372
- WO-A2-2016/149653
- WO-A2-2019/102269
- US-A1- 2019 076 152
- US-A1- 2019 083 102
- US-A1- 2019 105 057

## Description

### Field of the Invention

The present disclosure generally relates to the field of occlusion devices for the temporary occlusion of a blood vessel. More specifically, the disclosure relates to Resuscitative Endovascular Balloon Occlusion of the Aorta (REBOA) via a unique occlusion assembly and method of employing the same.

### Background of the Invention

Devices utilized in REBOA procedures are generally occlusion catheters that are inserted through the groin and advanced into the aorta, where the occlusion assembly, such as a balloon, is expanded in order to occlude the aorta thereby cutting off or reducing blood flow to organs downstream from the balloon and thereby increasing blood flow above or upstream of the balloon, specifically to the heart and the brain.

Preferably, catheters used in REBOA techniques should have as low a profile as possible so as to minimize complications during insertion, particularly those that are associated with the risk of bleeding when accessing arteries. Known REBOA devices have profiles which allow them to be inserted via relatively larger introducer sheaths of between 7 and 12 French. A lower profile would allow for easier insertion of the device since a smaller access hole in an artery will suffice. In turn, this may reduce or eliminate the need for large sheaths to guide entry. Furthermore, removal of a device having a lower profile may also reduce the risk of bleeding, since a smaller access hole also leads to reduced bleeding from the access site, which is particularly important in a battlefield or emergency setting.

A low profile device with the addition of an atraumatic tip eliminates the need to be tracked over an initially placed endovascular guide wire. This offers other advantages as well, including ease of use with minimal training, and may dispense with the need of using imaging, such as by fluoroscopic or X-ray guidance to make sure that the balloon is in place before inflation and occlusion of an artery. This is especially beneficial in emergency settings, when the expert users and imaging equipment may not be available.

There remains a need among REBOA devices for an occlusion assembly that has as reduced a profile as is possible, capable of atraumatic insertion, and does not require tracking over an initially placed endovascular wire, that may be utilized in a variety of conditions by personnel ranging from trained physicians in a hospital setting, to first responders in an emergency or battlefield setting. We have discovered that a REBOA device should be capable of smooth transitional inflation and deflation to ensure proper occlusion of the aorta during use, while providing various degrees of partial occlusion of the aorta to allow transient flow past the balloon to the ischemic tissues. We have also discovered that the ability to overinflate the balloon with a reduced risk of balloon or blood vessel rupture is desirable in some instances as it permits safe usage and facilitates placement in emergency settings.

The occlusion assembly disclosed herein, meets all of the needs mentioned above in a single device.

In the background art, WO 2020/033372 A1 discloses an occlusion catheter system for full or partial occlusion of a vessel having a vessel diameter. The occlusion catheter system includes a proximal catheter shaft having a proximal lumen and a hypotube positioned partially within the proximal lumen and spaced from the proximal catheter shaft. The catheter system also includes a distal catheter shaft attached to a distal end of the hypotube and an occlusion balloon connected at a proximal end to the proximal catheter shaft and at a distal end to the distal catheter shaft. The occlusion balloon is configured to define flow channels with inner surfaces of the vessel at folds in the occlusion balloon when the occlusion balloon is partially inflated and in engagement with the inner surfaces.

US 2019/083102 A1 discloses examples of aortic occlusion devices that include radially expandable and collapsible proximal and distal end portions, such as annular self-expanding stents or frames, that are configured to radially expand within an aorta to secure the device within the aorta. The devices can also include a catheter extending axially between the distal end portion and the proximal end portion and a porous covering, or filter, positioned around the catheter and between the proximal end portion and the distal end portion and configured to filter emboli from blood flowing into upper-body arteries. The device can further include a one-way valve positioned at or adjacent to the distal end portion of the device and configured to restrict retrograde blood flow through the device toward the heart.

US 2019/076152 A1 discloses an occlusion catheter system and includes an inflation catheter member and an occlusion balloon. The proximal and distal balloon ends are connected to the inflation catheter between the proximal and distal catheter ends. A distal pressure sensor is attached to the inflation catheter member between the proximal balloon end and the atraumatic tip. An inflatable spine is connected to the inflation catheter. The proximal spine end is connected to the inflation catheter near the proximal balloon end and the distal spine end is connected to the inflation catheter near the distal balloon end. The occlusion balloon and the inflatable spine are configured to define blood flow channels with the internal surface and the external balloon surface when the occlusion catheter system is at least partially positioned in the vessel and the occlusion balloon and the inflatable spine are in a partially inflated configuration.

### Summary of the Invention

The present invention is defined by claim 1. In contrast to conventional REBOA occlusion devices, the present device may be inserted into a patient via an introducer sheath having a lower profile as small as 4 French.

The device includes an atraumatic J-tip with a built in, peel-off, J-tip straightener that allows the atraumatic tip to be easily inserted into an introducer sheath.

The main components of the device are a single elastomeric molded balloon that envelopes a portion of the elongate shaft and its central wire. Proximal of the balloon envelope, the elongate shaft defines a longitudinal passage that does double duty as an inflation lumen and wire positioning lumen. Distal of the balloon envelope the elongate shaft is adhered to the wire. The balloon envelope shape is modified by stretching and bonding each end of the balloon envelope over a mounting region of the elongate shaft, which is itself constructed of two types of extruded polyether block amide (PEBA) materials. The elongate shaft has an inflation outlet port within the interior of the balloon envelope that is in fluid communication with the central passage. The central passage extends proximally along the length of the shaft to an inflation inlet port, into which inflation fluid for expanding the balloon envelope may be injected, via a syringe or other mechanism.

The balloon envelope has a pre-molded size and shape. This, along with its elastomeric construction and the manner of it being bonded to the mounting region of the elongate shaft, provides the balloon envelope with several operation modes, or states, of operation other than being limited to an unexpanded state and a fully expanded state.

In contrast to conventional spherical or rounded occlusion balloons, the balloon envelope of the type disclosed herein, has a generally "reverse tear drop" or "ice cream cone" shape. The essential sameness of the shap of the balloon envelope independent of the inflation volume over a range of operational states is referred to herein as a "self-similar shape". This general shape is largely maintained over the entire range of inflation states. The reproducibility of the shape at several inflation volumes allows the balloon envelope to form a variable valve with the descending aorta in operation. This attribute, in combination with the narrow profile of the inflation lumen, allows the device to address two important medical concerns. The first is the reduction of shock due to a too rapid restoration of flow when the device moves from a fully inflated state to the minimal, or uninflated, state. Reduction in shock makes the device much safer in use than prior devices. Secondly, the ability to operate at intermediate inflation values allows for physician control of limited and controlled perfusion distal to the balloon to support organs, thus extending the time that the device may be used to treat patients. This is a benefit in both emergency and clinical settings, and greatly improves the utility of the device in contrast to conventional devices offering only "on" and "off" flow states.

The balloon envelope of the present device may also be safely over inflated over its normal "fully inflated" state. This provides further utility over conventional REBOA devices. Over inflation of the balloon envelope with conventional REBOA devices can predispose the balloon envelope to damage and/or the aorta to rupture. The ability to overinflate the balloon envelope in the aorta is an important safety feature of the present device allowing a larger window of inflation volumes to the user to reduce the overall risk of inflation. When over inflated in an upside down Y-shaped vessel bifurcation, for example the aorto-iliac bifurcation, the balloon will essentially pull itself gently into the larger vessel. This reduces the risk of the balloon envelope rupturing the narrower iliac artery and instead the balloon envelope is gently pulled up into the wider aorta greatly facilitating ease of use and safety.

These and other attributes and embodiments of the present occlusion device are shown in the accompanying drawings and described in greater detail below.

### Brief Description of the Drawings

Fig.1 is an illustration of a region of human anatomy wherein an embodiment of the occlusion assembly is shown in use in a REBOA procedure.
Fig. 2 is a side view of the embodiment of the occlusion device, shown before use.
Fig. 3 is a cross-sectional view of the embodiment shown in Fig. 2 taken at line X.
Fig. 4 is a cross-sectional view of the embodiment shown in Fig. 2 taken at line Y.
Fig. 5 is a cross-sectional view of the embodiment shown in Fig. 2 taken at line Z.
Fig. 6 is a close-up view of the balloon envelope and the adjacent balloon mounting region of the occlusion assembly shown in Fig. 2, depicted in longitudinal sectional view.
Fig. 7 is a side view of a distal end portion of an embodiment of the occlusion device shown prior to the bonding of the pre-molded balloon envelope to the elongate shaft.
Fig. 8 is a longitudinal, sectional view, of the distal end portion of the occlusion device shown in Fig. 7 but in an assembled state and the balloon envelope inflated to its normal or fully inflated state in an unconfined environment.
Fig. 9 is a longitudinal, sectional view, of the distal end portion of the embodiment shown in Fig. 8, with the balloon envelope in the fully inflated state and occluding the aorta of a patient.
Fig. 10 is a longitudinal, sectional view, of the distal end portion of the embodiment shown in Fig. 9, shown during initial inflation of the balloon envelope.
Fig. 11 is a longitudinal, sectional view, of the distal end portion of the embodiment shown in Fig. 10, with the balloon envelope depicted in a partially inflated state.
Fig. 12 is a longitudinal, sectional view, of the distal end portion of the embodiment shown in Fig. 11 with the balloon envelope in an over inflated.
Fig. 13 is a is a longitudinal, sectional view, of the distal end portion of the embodiment shown in Fig. 12 wherein the balloon in the over inflated state is drawn distally into a side vessel of a bifurcation.
Fig. 14 is a side view of the embodiment shown in Fig. 2 shown in a fully assembled state and equipped with a side-arm shaft assembly, J-tip straightener and inflation syringe.
Fig. 15 is a close-up perspective view of the distal end of the embodiment shown in Fig. 14.
Fig. 16 shows the embodiment depicted in Fig. 15, with the J-tip straightener partially peeled off to illustrate the manner in which the atraumatic tip is straightened for ease of insertion into an introducer sheath (not shown).
Fig. 17 is a graph produced from experimental observation and measurements.
Fig. 18 is a graph produced from experimental observation and measurements.

### Detailed Description

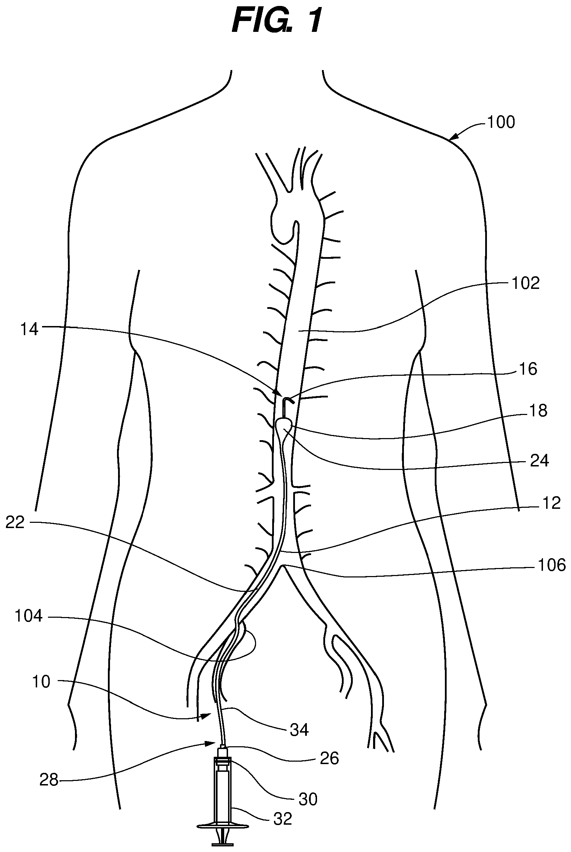

As indicated above, embodiments of the present invention are directed to an occlusion assembly 10 for use in REBOA procedures. An example of an embodiment of the occlusion assembly 10 as it may be used in a REBOA procedure is illustrated in Fig. 1. As is shown, the assembly 10 includes an elongate shaft 12, which terminates at a distal shaft end 14 in an atraumatic J-tip 16. Proximal to the J-tip 16 is positioned a balloon envelope 18. The shaft 12 defines a central passage or lumen 22, which is in fluid communication with the interior 24 of the balloon envelope 18 and sealed at the distal end of the balloon. The lumen 22 extends from the balloon envelope 18 to an inflation port 26 located at the proximal shaft end 28. Through the inflation port 26, inflation fluid 30 may be injected into the lumen 22, and thus, the balloon envelope 18, via a syringe 32 or equivalent mechanism; thereby causing the balloon envelope 18 to expand and occlude the aorta 102.

During the REBOA procedure, the occlusion device 10 is advanced to a target site within the aorta 102 of a human patient 100. Though the occlusion assembly 10 may be inserted into the aorta 100 using a variety of different arterial pathways, in the embodiment shown, the occlusion assembly 10 is inserted initially into the femoral artery 104 via a 4 Fr introducer sheath (not shown), and then advanced into the aorta 102 beyond the aortic bifurcation 106. Once the balloon envelope 18 is at the target position within the aorta or other branch vessel, the balloon envelope 18 is expanded in the manner described above.

In at least one embodiment, proper positioning of the balloon envelope 18 may be visually estimated by way of one or more visual markers placed on the external surface of the elongate shaft 12. Such a marker 34, corresponds to the length of the elongate shaft 12, or the distance that the elongate shaft must be advanced into the aorta 102, in order to place the balloon envelope 18 in a desired anatomical area or zone. For example, in at least one embodiment, the assembly has a visual marker 34 corresponding to a length/distance of at least 40 cm from the balloon envelope 18 to the mark 34, which corresponds to a placement of the balloon envelope above the junction of the lowest renal artery in most adult patients.

Embodiments of the assembly 10 may have any number of visual markers to indicate proper deployment distances for specific anatomical positioning. In at least one embodiment, the elongate shaft has two visual markers 34, with one corresponding to a length/distance of 48 cm from the center of the balloon envelope 18 and the other corresponding to a length of 28 cm from the center of the balloon envelope 18. These marker designations correspond to Zone 1 of the thoracic aorta and Zone 3 of the infrarenal aorta.

In at least one embodiment, the visual marker(s) 34 may be customized to the assembly 10 based on pre-use examination of the patient.

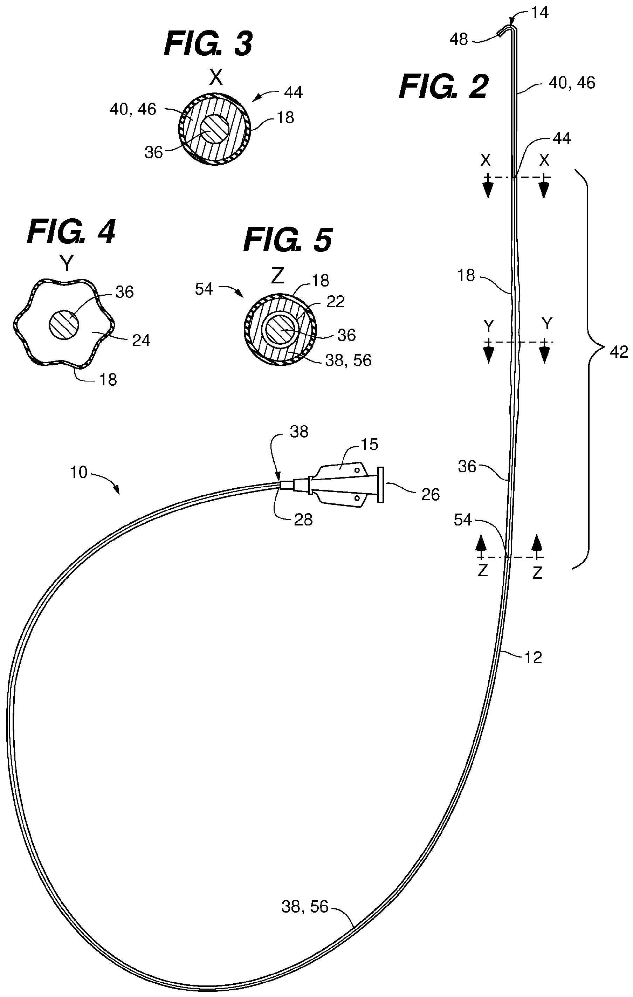

Turning to Fig. 2, an embodiment of an occlusion assembly 10 is shown prior to use. In this view, the presence of a support wire 36 is shown. The support wire or wire 36, is a stainless steel, or equivalent material, wire having a diameter of 0.03 inch (0.76 mm), or less, that extends the entire length of the elongate shaft 12 between the proximal shaft end 28 and the distal shaft end 14. In at least one embodiment, the wire has a diameter of 0.028 inch (0.71 mm). The length of the shaft 12 and the wire 36 is measured from the proximal shaft end 28 to the distal shaft end 14.

At the proximal shaft end 28, a hub 15 is engaged to the elongate shaft 12. The wire 36 is held in place relative to the other components of the elongate shaft 12 (said components are identified and discussed in more detail below) as well as the balloon envelope 18, by way of its proximal end 29 being embedded or other secured to the hub 15. The hub 15 also defines the inflation port 26, referenced in Fig. 1, which is in fluid communication with the inflation lumen 22 of the elongate shaft 12, and to which a syringe 32 or other inflation device may interface with the inflation lumen 22.

At the opposite end of the assembly 10, the wire 36 terminates at the atraumatic J-tip 16. The J-tip 16 is a 5 cm coil or J-curve of approximately 180 to 360 degrees imparted to the wire 36 to ensure that the distal shaft end 14 does not catch or otherwise harm the vessels through which the occlusion assembly 10 is advanced.

In at least one embodiment, the length of the elongate shaft 12 is no greater than 75 cm. In at least one embodiment the length of the elongate shaft is no greater than 65 cm. If the access site is at another area, such as the radial artery in the wrist, then the length of the elongate shaft is no greater than 85 cm. In another embodiment for pediatric patients, the elongate shaft is no longer than 45 cm.

In Fig. 2, three cross-sectional reference lines X, Y and Z are labeled at different points along the length of the elongate shaft 12. These cross-sections are depicted in FIGs. 3, 4 and 5 respectively, and illustrate the manner in which the balloon envelope 18 is bonded or welded to the materials of the elongate shaft 12.

Note that in the embodiment illustrated in in Figs. 3-6, the bonding or welding of the balloon envelope 18 to the elongate shaft 12 is, for purposes of illustration and description, presented to show the relevant structures in overlapping engagement, it will be understood by those of ordinary skill in the art that the relevant structures may alternatively be bonded or welded together end to end (i.e. butt welded or joined).

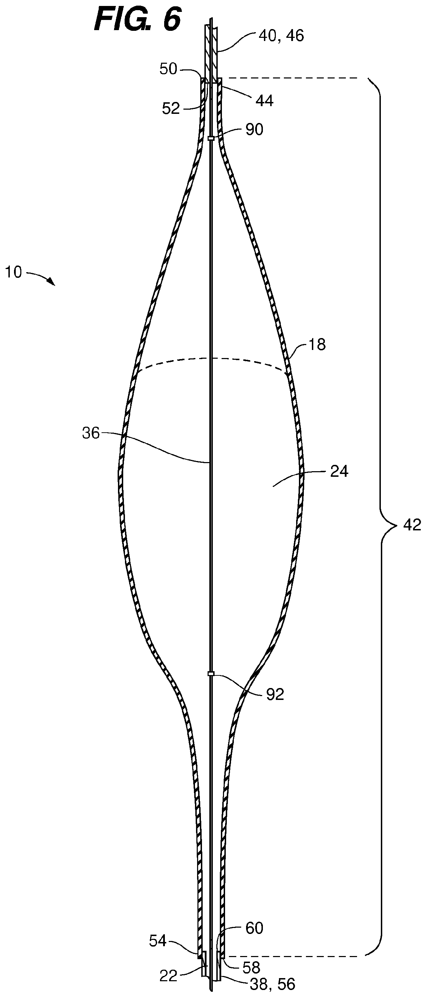

The reference lines X, Y and Z are also useful for dividing up the elongate shaft into three component regions that make up the elongate shaft 12. Extending distally from the proximal shaft end 28 to cross-sectional reference Z, the elongate shaft 12 comprises a proximal shaft region 38. Extending distally from cross-sectional reference X to the distal shaft end 14, the elongate shaft 12 comprises a distal shaft region 40. Extending between the proximal shaft region 38 and the distal shaft region 40 (i.e. between cross-sectional reference Z and cross-sectional reference X), the elongate shaft 12 comprises a balloon mounting region 42, directly visible in Fig. 6, but obscured here by the presence of the balloon envelope 18, which is mounted over the balloon mounting region 42.

Turning now to the cross-sectional views depicted in Figs. 3, 4, and 5, in Fig. 3, a section of the elongate shaft 12 is shown which corresponds to a distal bonding region 44. The distal bonding region 44 marks the distal end of the balloon mounting region 42 and the beginning of the distally extending distal shaft region 40. The distal shaft region 40 comprises a layer 46 of polyether block amide (PEBA) that is extruded on to a distal portion of the wire 36 extending from the distal bonding region 44 to the terminal end 48 of the atraumatic J-tip 16. A distal end 50 of the balloon envelope 18 is bonded or welded to the proximal end 52 of the PEBA layer 46 of the distal shaft region 40. PEBA layer 46 adheres to the wire 36 thus sealing both the distal shaft region 40 and the distal end 50 of the balloon envelope 18 to the wire 36.

In at least one embodiment, the PEBA layer 46 of the distal shaft region 40 is a lubricious form of PEBA sold under the trademarked name VESTAMIDE^{®} EVERGLIDE^{®} MED by the Polymer Dynamix company.

In at least one embodiment the distal shaft region 40 and the corresponding layer 46 have a length of no greater than 8 cm as measured from the distal bonding region 44 to the terminal end 48 of the atraumatic J-tip 16.

Skipping Fig. 4 for the moment, and looking now to Fig. 5, depicted in Fig. 5 a section of the elongate shaft 12 is shown, which corresponds to the position of a proximal bonding region 54.

The proximal bonding region 54 marks the proximal end of the balloon mounting region 42 and the beginning of the proximally extending proximal shaft region 38. The proximal shaft region 38 comprises a tube 56 of polyether block amide (PEBA) that is disposed about that portion of the wire 36 extending from the proximal bonding region 54 to the proximal shaft end 28. The tube 56 defines the inflation lumen 22, which does double duty as a passage through which the wire 36 extends. A proximal end 58 of the balloon envelope 18 is bonded or welded to the distal end 60 of the tube 56 at the proximal bonding region 54. The distal end 60 of the tube 56 corresponds with the end of the inflation lumen 22, which is in fluid communication with the interior 24 of the balloon envelope 18.

In at least one embodiment the tube 56 is manufactured from a form of PEBA sold under the trademark PEBAX^{®} and manufactured by the Compounding Solutions company.

Returning now to Fig. 4, the cross-section depicted in Fig 4 is a section of the assembly 10 corresponding approximately to the mid-point of the working portion of the balloon envelope 18. As is shown, the balloon envelope 18 is disposed about the wire 36, with the bare wire 36 passing through the interior 24 of the balloon envelope 18, between the proximal bonding region 54 and the distal bonding region 44.

In at least one embodiment, the balloon envelope is formed from an elastomeric polymer such as Urethane.

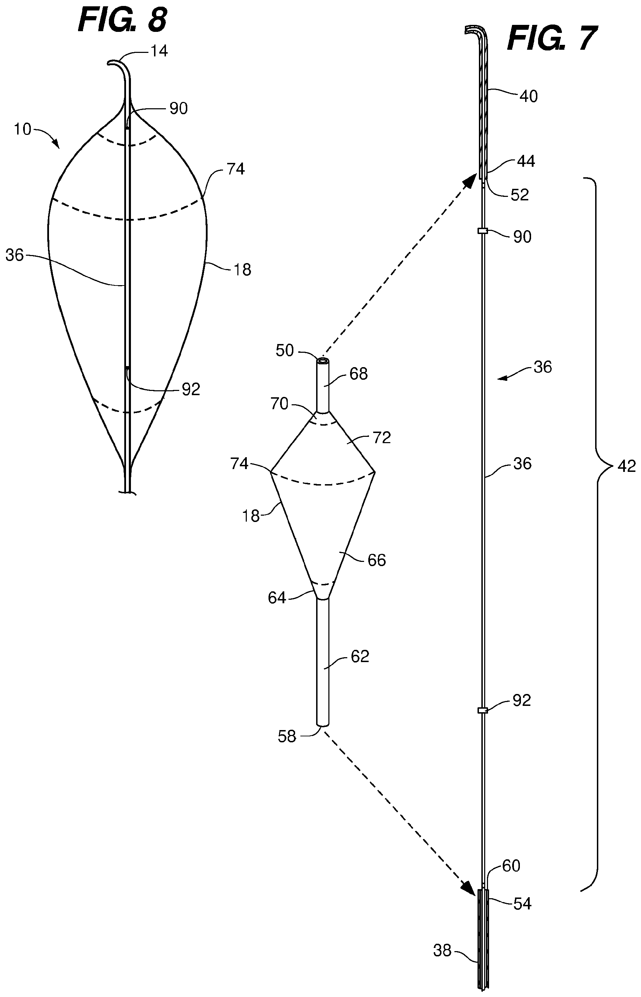

Turning to Fig. 7 the elongate shaft 12 is shown adjacent to the balloon envelope 18 prior to the balloon envelope being mounted onto the elongate shaft 12. The balloon envelope 18 as shown in its as molded state. The balloon envelope 18 is a contiguous envelope of elastomeric material which is molded into form whose working portion has a shape akin to an ice cream cone or reverse tear drop. This shape has several identifiable portions that are useful in describing the balloon and its performance characteristics. Starting from the proximal end 58, the balloon envelope 18 includes a proximal neck 62, which transitions into a conical proximal shoulder section 64; this shoulder section transitions into a conical proximal taper section 66. From the distal end 50 the balloon envelope 18 includes a distal neck 68, which transitions into a distal shoulder section 70; this shoulder section transitions into a truncated conical distal blunt section 72.

As is shown, conical proximal taper section 66 and truncated conical distal blunt section 72 intersect at a meridian 74, which marks the area of the balloon envelope 18 having the largest as molded diameter. In the as molded state, the conical proximal taper section 66 has a greater longitudinal length than that of the truncated conical distal blunt section 72.

In at least one embodiment, the balloon envelope 18, in the molded state has a total length of approximately 70 mm as measured from the proximal end 58 to the distal end 50, and an outer diameter of approximately 8 mm at the meridian 74. The proximal neck 62 has a length of approximately 10 cm and the distal neck 68 has an approximate length of 2 cm and both have a contiguous outer diameter of approximately 1.35 mm.

As is shown in Fig. 7, the balloon mounting region 42 has a length greater than the length of the balloon envelope itself. Thus, the balloon envelope 18 must be longitudinally stretched in order to be mounted onto the elongate shaft 12. In at least one embodiment, the balloon mounting region is at least 2.5 cm longer than the balloon envelope 18.

The dimensions and shape of the balloon envelope 18, in combination with the unique construction of the elongate shaft 12, not only allows for the occlusion assembly to be inserted into the patient using an introducer sheath as small as 4 Fr, but also allows the balloon envelope 18 to have multiple useful inflation states and unique inflation characteristics.

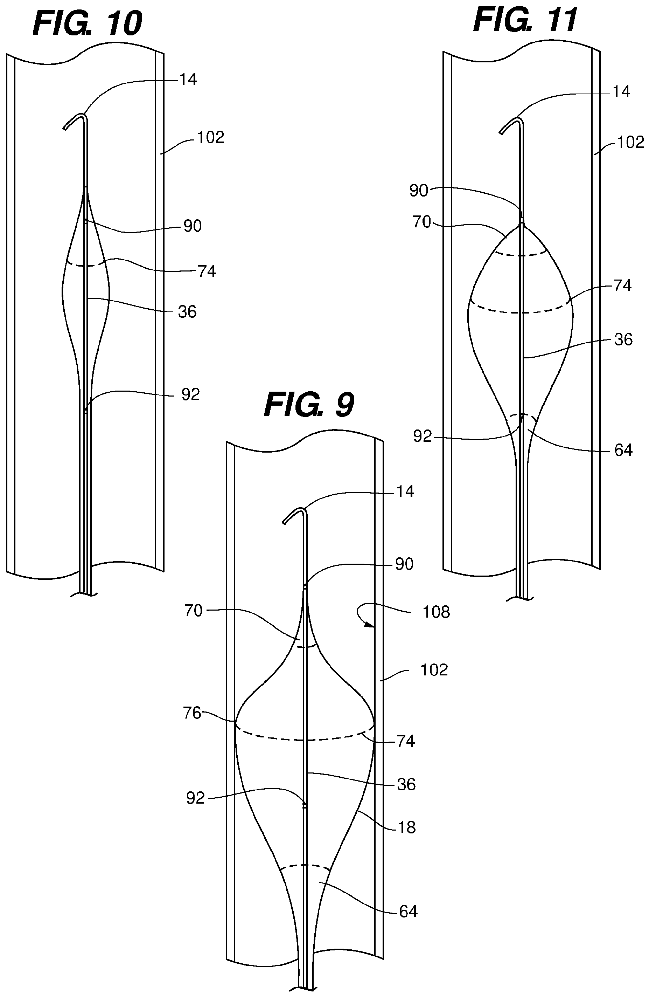

For purposes of standard REBOA use, the balloon envelope 18 has a fully inflated state, such that when the balloon envelope 18 is fully expanded, the meridian 74 will correspond with that region of the envelope 18 having the greatest diameter, such as in the manner illustrated in Figs. 8 and 9. This relationship of the meridian's position as the widest section of the balloon envelope 18 is constant whether the envelope 18 is expanded to its fully inflated state within the confines of the aorta 102 and subject to blood pressure acting against it, such as in the depiction of Fig. 9; as well as when the balloon envelope 18 is expanded to its fully inflated state outside of the body, such as in the depiction of Fig. 8.

In at least one embodiment, the fully inflated state of the balloon envelope is achieved by injection of between 10-15 ccs of inflation fluid (e.g. saline) into the interior of the balloon envelope in the manner previously described. When fully expanded the meridian 74 has an outer diameter of approximately 25-30 mm. When positioned within the aorta 102, and inflated to the fully inflated state, such as in the manner shown in Fig. 9, the external surface 76 of the balloon envelope 18 should be in contact with the vessel wall 108 and providing complete occlusion to blood flow.

As implied above, the position of the meridian 74 is not constant in the various inflation states. For example during initial inflation, i.e. a low inflation state such as is shown in Fig, 10, or a partial inflation state such as is shown in Fig. 11, the meridian 74 has a first diameter and is located at a first distance from the distal shaft end 14. But as the balloon envelope 18 reaches the fully inflated state shown in Fig. 9, the meridian 74 has a larger diameter than in the other states and has transitioned further away from the distal shaft end 14.

Other aspects of the balloon envelope 18 will vary during expansion as well. For example, as the balloon envelope 18 is expanded from the partial inflation state of Fig. 11 to the fully inflated state of Fig. 9, the volume of the conical distal shoulder section 70 decreases such that in the fully expanded state the conical distal shoulder section volume is less than the conical distal shoulder section volume in the partially inflated state. Whereas the opposite occurs in the conical proximal shoulder section 64. In that section of the balloon envelope 18, the volume increases as the balloon envelope is expanded, such that in the fully inflated state the conical proximal shoulder section volume is greater than the conical proximal shoulder section volume in the partially inflated state.

During inflation, as the balloon envelope 18 is acted upon more and more by the blood pressure, the balloon envelope 18 migrates downwards along the wire 36 (in the direction of the proximal shaft end) and then eventually catches the aortic wall 108 for full occlusion. When the balloon envelope 18 is deflated, and blood is allowed to pass around the external surface of the balloon 76, the balloon envelope 18 will begin to go back to its unmigrated position.

With the offset nature of the balloon envelope 18 and also because of the stretch imparted to the balloon envelope 18 when it is mounted onto the balloon mounting region 42 (2.5 cm in at least one embodiment as discussed above), the general shape of the balloon envelope 18 is maintained during the inflation and deflation processes, which allows the fine adjustments (titratability) of blood flow. This is in contrast to known spherical balloons that are fixed to a catheter shaft and imparted with no stretch. Such balloons are unable to migrate and therefore the shape of the balloon changes substantially when acted on by blood pressure. Such balloons act much like an on/off switch in terms of performance (i.e. no appreciable occlusive effect before full occlusion at full inflation) and do not provide for the ability to be adjusted in the manner of the present balloon envelope 18 nor have the ability to gradually recirculate blood flow in a graduated manner during deflation such as the present device 10 provides.

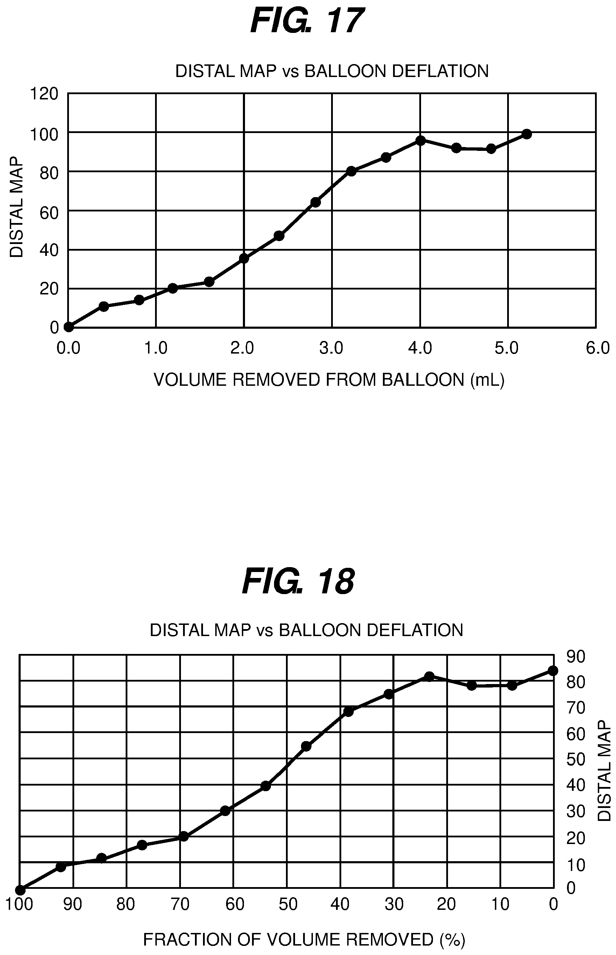

The ability of the balloon envelope 18, and the device 10, to provide gradual and incremental occlusive effects is illustrated in Figs. 17 and 18.

Fig. 17 and Fig. 18 should be considered together. They are plots of data taken in vivo from a porcine model used in development of the device 10. As mentioned above, in the prior art the occlusion balloon is either in the fully occluded state or effectively in the fully contracted state permitting full flow past the device. In contrast to this, the present device 10 provides for proportionate flow permitted past the balloon envelope 18 prior to and following full occlusion.

This is demonstrated by occluding the aorta and measuring the Mean Arterial Pressure (MAP) distal of the balloon. The measured MAP is seen on the Y-Axis in both Fig. 17 and Fig. 18. Referring to Fig. 17 note that the relationship between measured pressure and balloon envelope volume is nearly linear over the range of slightly less than 1 ml taken from the balloon envelope to the point where 4 ml are removed. Procedurally this corresponds to reducing volume in the balloon envelope from total occlusion to near total deflation. The linearity demonstrates that the pressure driving flow in the organ increases in a gradual fashion as balloon envelope volume is slowly reduced.

In Fig. 18 the same information is expressed as a function of percent balloon envelope volume reduction. Qualitatively this shows that inflation volume controls MAP over the full range of values from total occlusion to maximal flow. The self-similar shape attribute of the balloon envelope is maintained over a substantially linear range of inflation volume. This graphed data demonstrates that the amount of blood flow past the balloon envelope will be proportional to inflation volume over the operational range defined by the range of the self-similar shape of the balloon.

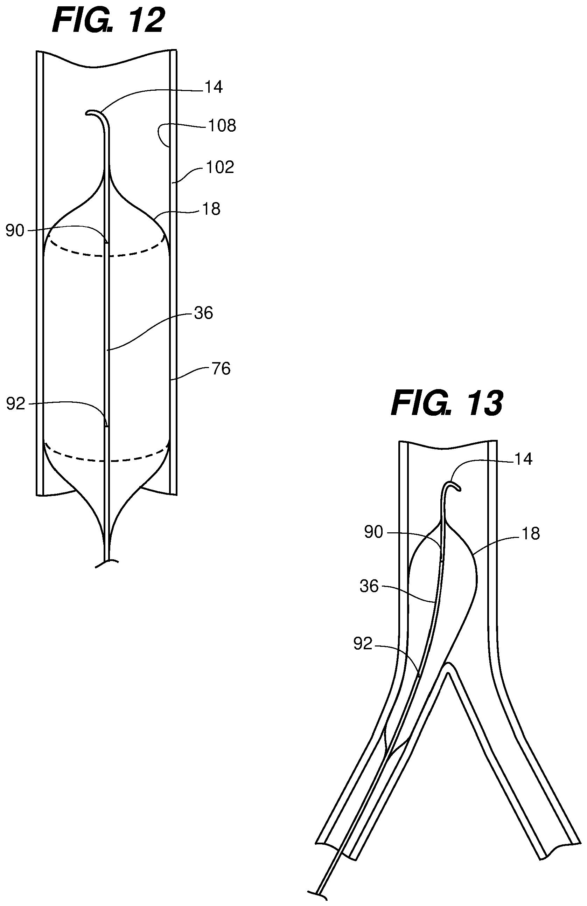

A unique feature of the present assembly 10, is the capacity to safely inflate the balloon envelope 18 into an over inflated state such as is shown in Figs. 12 and 13. When over inflating the balloon 18 within the aorta 102 beyond the fully inflated state, the meridian 74 does not further increase in diameter from that in the fully inflated state, but rather will increase in longitudinal length, effectively transitioning the meridian from a narrow band of intersection with the aortic wall 108, to potentially the majority of the balloon envelope's external surface 76 being in contact with the wall 108. This is accomplished as a result of the shoulder sections 64 and 70 growing, rather than as a result of further stretching by the conical proximal taper section 66 and truncated conical distal blunt section 72.

This longitudinal widening of the meridian 74 is accompanied by a longitudinal advancement/growth of the balloon envelope 18 such that in the over inflated state the meridian 74 is closer to the distal shaft end 14 than in the fully inflated state, the partially inflated state, or the low inflation state.

There is an additional benefit of preventing vessel damage at a bifurcation with the ability to advance the balloon envelope 18 distally via the "growth" of the shoulder sections 64 and 70. For example, as shown in Fig. 13, when the balloon envelope 18 is over inflated in a upside-down Y-shaped vessel bifurcation (aorto-iliac bifurcation), the balloon will pull itself gently into the larger vessel (aorta) and prevent damage to the smaller vessel (iliac artery). The ice-cream cone shape of the balloon envelope 18 also promotes this growth into the larger vessel by preferentially inflating the wider ice-cream cone section of the truncated conical distal blunt section 72 of the balloon envelope 18, as long as this portion of the balloon is above the bifurcation.

In some embodiments, the balloon envelope 18 may be over inflated up to 700% by volume over the fully inflated state. A key characteristic of the present assembly 10, is that regardless of the degree of over inflation when properly used in the manner described herein, the balloon envelope will fail before damaging the aorta.

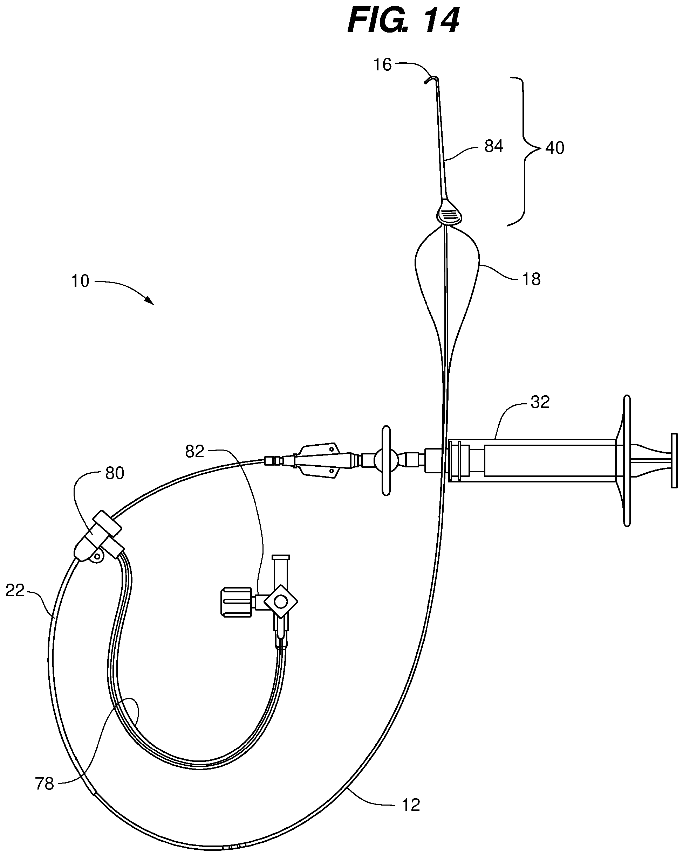

In addition to the characteristics discussed thus far, embodiments of the occlusion assembly 10 disclosed herein are provided with several other features that benefit both safety and ease of use during a REBOA procedure, an example of such an embodiment is shown in Fig. 14.

In the embodiment shown, the assembly 10, is provided with a side-arm shaft assembly 78 which is in fluid communication with the inflation lumen 22 of the elongate shaft 12 via a t-valve 80. The side-arm shaft assembly 78 includes a stop cock valve 82 that may be open and shut to allow inflation fluid to egress from the lumen 22 and provides the user with greater control of the inflation and deflation of the balloon envelope that a syringe 32 may allow by itself. The side-arm assembly may also act as an interface for a blood pressure monitor.

In the present embodiment, the assembly 10 is also provided with a J-tip straightener 84 that is preloaded over a portion of distal shaft region 40, between the distal end 50 of the balloon envelope 18 and the atraumatic J-tip 16.

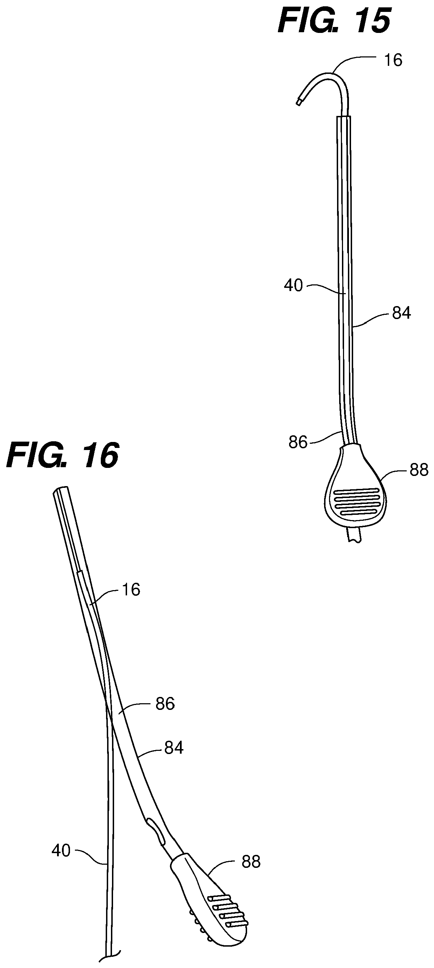

The J-tip straightener 84 has a unique construction and role as illustrated in more detailed views of Figs. 15 and 16. The J-tip straightener is essentially a hollow peel-off shaft or tube 86 that is disposed about a portion of distal shaft region 40. When the assembly 10 is ready for use, the user slides the J-tip straightener 84 onto and over the coil of the atraumatic J-tip 16, which will temporarily straighten as a consequence of the confinement and advancement of the J-tip straightener 84 over the tip 16. This temporary straightening of the atraumatic J-tip 16 allows it to be more easily threaded into the 4 Fr introducer (not shown) during initial insertion of the assembly during a REBOA procedure.

For further ease of operation the J-tip straightener 84 includes a user engagement tab or grip 88 that protrudes from the peel-off shaft 86, and which user may grasp and pull distally to more easily advance of the J-tip straightener 84 over the J-tip 16.

Finally, in at least some embodiments, the elongate shaft 12 is provided with at least two radiopaque (RO) markers 90 and 92, such as are shown in Figs. 6-13. As illustrated, the RO markers 90 and 92 are placed on the wire 36 at locations within the balloon mounting region 42 so as to allow the position of the balloon envelope 18 to be monitored within the patient, via a visualization mechanism (Fluoroscope, etc.), during the performance of a REBOA procedure utilizing the assembly 10.

The many features and advantages of the invention are apparent from the above description. Numerous modifications and variations will readily occur to those skilled in the art. Since such modifications are possible, the invention is not to be limited to the exact construction and operation illustrated and described. Rather, the present invention should be limited only by the following claims.

As used herein terms such as "about" or "approximately" and the like when used to describe a measurement value attributed to any aspect of the occlusion assembly 10, or any of its components, such terms are provided so as to reflect the range of tolerances inherent in the production of a given article of manufacture or its assembly as understood by one of ordinary skill.

## Claims

1. An occlusion assembly (10) for occluding the aorta of a patient, comprising:
(i) a balloon envelope (18), the balloon envelope constructed from an elastomeric material, the balloon envelope defining an interior (24) and an exterior (76), the balloon envelope having an as molded state, in the as molded state the balloon envelope has an ice cream cone shape, the balloon envelope having a longitudinal length extending between a proximal end (58) and a distal end (50) of the balloon envelope,
a proximal tubular neck (62) extends distally from the proximal end of the balloon envelope to a conical proximal shoulder section (64), a conical proximal taper section (66) extends distally from the conical proximal shoulder section,
a truncated conical distal blunt section (72) extends distally from the conical proximal taper section and forms a juncture therewith,
a conical distal shoulder (70) section extends distally from the truncated conical distal blunt section to a distal tubular neck (68), the distal tubular neck terminating at the distal end of the balloon envelope,
the juncture between the truncated conical distal blunt section and the conical proximal taper section defining a meridian (74), in the as molded state the meridian of the balloon envelope having a greater diameter than adjacent sections of the balloon envelope and the conical proximal taper section having a greater longitudinal length than that of the truncated conical distal blunt section; and
(ii) an elongate shaft (12), the elongate shaft having a proximal shaft end (28), a distal shaft end (14), and a shaft length extending therebetween, the elongate shaft comprising:
a wire (36), a proximal shaft region (38) of a first material, a distal shaft region (40) of a second material, and a balloon mounting region (42) extending between the proximal shaft region and the distal shaft region,
the wire extending the length of the elongate shaft with portions of the wire extending through the proximal shaft region, the balloon mounting region and the distal shaft region,
the proximal shaft region defining an inflation lumen (22) that extends from the proximal shaft end to the balloon mounting region, the portion of the wire extending through the proximal shaft region is positioned within the inflation lumen, the portion of the wire extending through the distal shaft region is adheringly engaged to the distal shaft region,
the balloon mounting region having a length greater than the length of the balloon envelope in the molded state, the length of the balloon mounting region extending from a proximal bonding region (54) located on a distal end of the proximal shaft region to a distal bonding region (44) located on a proximal end of the distal shaft region;
the proximal end of the balloon envelope is bonded to the proximal bonding region and the distal end of the balloon envelope is bonded to the distal bonding region,
the balloon envelope bonded to the elongate shaft has a longitudinally stretched state, wherein the balloon envelope is configured to be longitudinally stretched in order to be mounted onto the elongate shaft, wherein the length of the balloon envelope corresponds to the length of the balloon mounting region,
the interior of the balloon envelope is in fluid communication with the inflation lumen; and
(iii) a proximal hub (15), the proximal shaft end is engaged to the hub and extends proximally therefrom, the hub defining an inflation port (26), the inflation port is in fluid communication with the inflation lumen,
wherein the wire has a proximal tip, the proximal tip is fixedly engaged to the hub.

2. The assembly of claim 1, wherein the balloon envelope bonded to the elongate shaft is inflatably transitional from and between a low inflation state, a partial inflation state, a fully inflated state, and an overinflated state,
the conical distal shoulder section defines a conical distal shoulder section volume, the conical proximal shoulder section defines a conical proximal shoulder section volume, in the fully inflated state the conical distal shoulder section volume is less than the conical distal shoulder section volume in the partially inflated state, in the fully inflated state the conical proximal shoulder section volume is greater than the conical proximal shoulder section volume in the partially inflated state.

3. The assembly of claim 2, wherein in the partial inflation state the meridian has a first diameter and is located at a first distance from the distal shaft end, in the fully inflated state the meridian has a second diameter greater than the first diameter and is located at a second distance from the distal shaft end, the second distance is greater than the first distance, optionally
wherein in the overinflated state the meridian maintains the same diameter as in the inflated state, the meridian is located at a third distance from the distal end of the balloon envelope, the third distance is less than the second distance.

4. The assembly of claim 1, further comprising:
a first radiopaque marker (90) and a second radiopaque marker (92), the first radiopaque marker is engaged to the wire at a position distal to the proximal bonding region, and the second radiopaque marker is engaged to the wire at a position proximal to the distal bonding region.

5. The assembly of claim 1, wherein the length of the elongate shaft is not greater than 75 cm.

6. The assembly of claim 5, wherein the distal shaft region has a distal shaft region length of not greater than 8 cm, the distal shaft region defining an atraumatic J-tip (16) at the distal shaft end, the atraumatic J-tip having a length of not greater than 5 cm.

7. The assembly of claim 6, further comprising a J-tip straightener, the J-tip straightener comprises a peel-off shaft and a user engagement tab affixed to the peel-off shaft, the peel-off shaft slidingly engaged to and removeably disposed about a portion the distal shaft region between the atraumatic J-tip and the distal end of the balloon envelope.

8. The assembly of claim 1, wherein the first material and the second material are both extruded polyether block amide, the first material having different material properties than the second material.

9. The assembly of claim 8, wherein the first material is PEBAX^{®} and the second material is VESTAMIDE^{®} EVERGLIDE^{®} MED, and the elastomeric material is Urethane.

10. The assembly of claim 1, wherein the longitudinal length of the balloon envelope in the as molded state is approximately 70mm and the diameter of the meridian is approximately 8mm, the distal neck having a length of approximately 2 cm and the proximal neck having a length of approximately 10 cm, in the fully inflated state the diameter of the meridian is between about 20mm and about 30mm.

11. The assembly of claim 1, having an operational diameter of no more than 4 Fr.

12. The assembly of claim 1, further comprising visual markers, the visual markers located at specific areas on an external surface of the proximal shaft region, each of the visual markers indicating the distance the balloon envelope must be advanced into the aorta in order to be positioned at an anatomical zone corresponding to each of the visual markers.

13. The assembly of claim 1, wherein the ice crème cone shape of the balloon envelope is a self-similar shape.

## Patentansprüche

1. Okklusionsanordnung (10) zum Okkludieren der Aorta eines Patienten, umfassend:
(i) eine Ballonhülle (18), wobei die Ballonhülle aus einem Elastomermaterial hergestellt ist, die Ballonhülle ein Inneres (24) und ein Äußeres (76) definiert, die Ballonhülle einen wie geformten Zustand aufweist, die Ballonhülle in dem wie geformten Zustand eine Eistütenform aufweist, die Ballonhülle eine Länge in Längsrichtung aufweist, die sich zwischen einem proximalen Ende (58) und einem distalen Ende (50) der Ballonhülle erstreckt,
wobei sich ein proximaler röhrenförmiger Hals (62) von dem proximalen Ende der Ballonhülle zu einem kegelförmigen proximalen Absatzabschnitt (64) distal erstreckt und sich ein kegelförmiger proximaler Konusabschnitt (66) von dem kegelförmigen proximalen Absatzabschnitt distal erstreckt,
wobei sich ein kegelstumpfförmiger distaler stumpfer Abschnitt (72) von dem kegelförmigen proximalen Konusabschnitt distal erstreckt und eine Verbindungsstelle damit bildet,
wobei sich ein kegelförmiger distaler Absatzabschnitt (70) von dem kegelstumpfförmigen distalen stumpfen Abschnitt zu einem distalen röhrenförmigen Hals (68) distal erstreckt, wobei der distale röhrenförmige Hals an dem distalen Ende der Ballonhülle endet,
wobei die Verbindungsstelle zwischen dem kegelstumpfförmigen distalen stumpfen Abschnitt und dem kegelförmigen proximalen Konusabschnitt einen Meridian (74) definiert, in dem wie geformten Zustand der Meridian der Ballonhülle einen größeren Durchmesser als benachbarte Abschnitte der Ballonhülle aufweist und der kegelförmige proximale Konusabschnitt eine größere Länge in Längsrichtung aufweist als diejenige des kegelstumpfförmigen distalen stumpfen Abschnitts; und
(ii) einen langgestreckten Schaft (12), wobei der langgestreckte Schaft ein proximales Schaftende (28), ein distales Schaftende (14) und eine sich dazwischen erstreckende Schaftlänge aufweist, wobei der langgestreckte Schaft Folgendes umfasst:
einen Draht (36), einen proximalen Schaftbereich (38) aus einem ersten Material, einen distalen Schaftbereich (40) aus einem zweiten Material und einen Ballonbefestigungsbereich (42), der sich zwischen dem proximalen Schaftbereich und dem distalen Schaftbereich erstreckt,
wobei sich der Draht über die Länge des langgestreckten Schafts erstreckt und sich Abschnitte des Drahts durch den proximalen Schaftbereich, den Ballonbefestigungsbereich und den distalen Schaftbereich erstrecken,
wobei der proximale Schaftbereich ein Aufblaslumen (22) definiert, das sich von dem proximalen Schaftende zu dem Ballonbefestigungsbereich erstreckt, der sich durch den proximalen Schaftbereich erstreckende Abschnitt des Drahts innerhalb des Aufblaslumens positioniert ist und der sich durch den distalen Schaftbereich erstreckende Abschnitt des Drahts sich mit dem distalen Schaftbereich haftend im Eingriff befindet,
wobei der Ballonbefestigungsbereich eine Länge aufweist, die größer als die Länge der Ballonhülle in dem geformten Zustand ist, und sich die Länge des Ballonbefestigungsbereichs von einem an einem distalen Ende des proximalen Schaftbereichs befindlichen proximalen Klebebereich (54) zu einem an einem proximalen Ende des distalen Schaftbereichs befindlichen distalen Klebebereich (44) erstreckt;
wobei das proximale Ende der Ballonhülle an den proximalen Klebebereich geklebt ist und das distale Ende der Ballonhülle an den distalen Klebebereich geklebt ist,
wobei die an den langgestreckten Schaft geklebte Ballonhülle einen in Längsrichtung gedehnten Zustand aufweist, wobei die Ballonhülle dazu konfiguriert ist, in Längsrichtung gedehnt zu werden, um auf dem langgestreckten Schaft befestigt zu werden, wobei die Länge der Ballonhülle der Länge des Ballonbefestigungsbereichs entspricht,
wobei das Innere der Ballonhülle mit dem Aufblaslumen in Fluidverbindung steht; und
(iii) eine proximale Nabe (15), wobei sich das proximale Schaftende mit der Nabe im Eingriff befindet und sich davon proximal erstreckt, die Nabe einen Aufblasport (26) definiert, der Aufblasport mit dem Aufblaslumen in Fluidverbindung steht,
wobei der Draht eine proximale Spitze aufweist und sich die proximale Spitze mit der Nabe feststehend im Eingriff befindet.

2. Anordnung nach Anspruch 1, wobei die an den langgestreckten Schaft geklebte Ballonhülle durch Aufblasen aus und zwischen einem wenig aufgeblasenen Zustand, einem teilweise aufgeblasenen Zustand, einem vollständig aufgeblasenen Zustand und einem übermäßig aufgeblasenen Zustand übergangsfähig ist,
wobei der kegelförmige distale Absatzabschnitt ein kegelförmiges distales Absatzabschnittvolumen definiert, der kegelförmige proximale Absatzabschnitt ein kegelförmiges proximales Absatzabschnittvolumen definiert, in dem vollständig aufgeblasenen Zustand das kegelförmige distale Absatzabschnittvolumen geringer ist als das kegelförmige distale Absatzabschnittvolumen in dem teilweise aufgeblasenen Zustand, wobei in dem vollständig aufgeblasenen Zustand das kegelförmige proximale Absatzabschnittvolumen größer ist als das kegelförmige proximale Absatzabschnittvolumen in dem teilweise aufgeblasenen Zustand ist.

3. Anordnung nach Anspruch 2, wobei in dem teilweise aufgeblasenen Zustand der Meridian einen ersten Durchmesser aufweist und sich in einem ersten Abstand von dem distalen Schaftende befindet, in dem vollständig aufgeblasenen Zustand der Meridian einen zweiten Durchmesser aufweist, der größer als der erste Durchmesser ist, und sich in einem zweiten Abstand von dem distalen Schaftende befindet, wobei der zweite Abstand größer ist als der erste Abstand, wobei optional
in dem übermäßig aufgeblasenen Zustand der Meridian den gleichen Durchmesser beibehält wie in dem aufgeblasenen Zustand, sich der Meridian in einem dritten Abstand von dem distalen Ende der Ballonhülle befindet, der dritte Abstand geringer ist als der zweite Abstand.

4. Anordnung nach Anspruch 1, ferner umfassend:
eine erste röntgendichte Markierung (90) und eine zweite röntgendichte Markierung (92), wobei sich die erste röntgendichte Markierung an einer Position distal des proximalen Klebebereichs mit dem Draht im Eingriff befindet und sich die zweite röntgendichte Markierung an einer Position proximal des distalen Klebebereichs mit dem Draht im Eingriff befindet.

5. Anordnung nach Anspruch 1, wobei die Länge des langgestreckten Schafts nicht mehr als 75 cm beträgt.

6. Anordnung nach Anspruch 5, wobei der distale Schaftbereich eine Länge des distalen Schaftbereichs von nicht mehr als 8 cm aufweist, der distale Schaftbereich eine atraumatische J-Spitze (16) an dem distalen Schaftende definiert und die atraumatische J-Spitze eine Länge von nicht mehr als 5 cm aufweist.

7. Anordnung nach Anspruch 6, ferner umfassend einen J-Spitzenstrecker, wobei der J-Spitzenstrecker einen abziehbaren Schaft und eine an dem abziehbaren Schaft angebrachte Benutzereingriffslasche umfasst, wobei sich der abziehbare Schaft gleitfähig mit einem Abschnitt des distalen Schaftbereichs zwischen der atraumatischen J-Spitze und dem distalen Ende der Ballonhülle im Eingriff befindet und entfernbar darum herum angeordnet ist.

8. Anordnung nach Anspruch 1, wobei das erste Material und das zweite Material beide extrudiertes Polyetherblockamid sind, wobei das erste Material andere Materialeigenschaften aufweist als das zweite Material.

9. Anordnung nach Anspruch 8, wobei das erste Material PEBAX^{®} ist und das zweite Material VESTAMIDE^{®} EVERGLIDE^{®} MED ist und das Elastomermaterial Urethan ist.

10. Anordnung nach Anspruch 1, wobei die Länge in Längsrichtung der Ballonhülle in dem wie geformten Zustand ungefähr 70 mm beträgt und der Durchmesser des Meridians ungefähr 8 mm beträgt, wobei der distale Hals eine Länge von ungefähr 2 cm aufweist und der proximale Hals eine Länge von ungefähr 10 cm aufweist, wobei in dem vollständig aufgeblasenen Zustand der Durchmesser des Meridians zwischen etwa 20 mm und etwa 30 mm beträgt.

11. Anordnung nach Anspruch 1, die einen Arbeitsdurchmesser von nicht mehr als 4 Ch aufweist.

12. Anordnung nach Anspruch 1, ferner umfassend visuelle Markierungen, wobei sich die visuellen Markierungen an spezifischen Gebieten auf einer Außenoberfläche des proximalen Schaftbereichs befinden, wobei jede der visuellen Markierungen die Strecke angibt, um die die Ballonhülle in die Aorta voranbewegt werden muss, um an einer jeder der visuellen Markierungen entsprechenden anatomischen Zone positioniert zu werden.

13. Anordnung nach Anspruch 1, wobei die Eistütenform der Ballonhülle eine selbstähnliche Form ist.

## Revendications

1. Ensemble d'occlusion (10) destiné à obturer l'aorte d'un patient, comprenant :
(i) une enveloppe de ballonnet (18), l'enveloppe de ballonnet construite à partir d'un matériau élastomère, l'enveloppe de ballonnet définissant un intérieur (24) et un extérieur (76), l'enveloppe de ballonnet ayant un état brut de moulage, à l'état brut de moulage l'enveloppe de ballonnet ayant une forme de cornet de glace, l'enveloppe de ballonnet ayant une longueur longitudinale s'étendant entre une extrémité proximale (58) et une extrémité distale (50) de l'enveloppe de ballonnet,
un col tubulaire proximal (62) s'étend de manière distale de l'extrémité proximale de l'enveloppe de ballonnet à une section d'épaulement proximale conique (64), une section effilée proximale conique (66) s'étend de manière distale depuis la section d'épaulement proximale conique,
une section émoussée distale conique tronquée (72) s'étend de manière distale depuis la section effilée proximale conique et forme une jonction avec celle-ci,
une section d'épaulement distale conique (70) s'étend de manière distale de la section émoussée distale conique tronquée à un col tubulaire distal (68), le col tubulaire distal se terminant au niveau de l'extrémité distale de l'enveloppe de ballonnet,
la jonction entre la section émoussée distale conique tronquée et la section effilée proximale conique définit un méridien (74), à l'état brut de moulage le méridien de l'enveloppe de ballonnet ayant un diamètre supérieur à celui de sections adjacentes de l'enveloppe de ballonnet et la section effilée proximale conique ayant une longueur longitudinale supérieure à celle de la section émoussée distale conique tronquée ; et
(ii) une tige allongée (12), la tige allongée ayant une extrémité de tige proximale (28), une extrémité de tige distale (14) et une longueur de tige s'étendant entre celles-ci, la tige allongée comprenant :
un fil (36), une région de tige proximale (38) d'un premier matériau, une région de tige distale (40) d'un deuxième matériau, et une région de montage de ballonnet (42) s'étendant entre la région de tige proximale et la région de tige distale,
le fil s'étendant sur la longueur de la tige allongée avec des parties du fil s'étendant à travers la région de tige proximale, la région de montage de ballonnet et la région de tige distale,
la région de tige proximale définissant une lumière de gonflage (22) qui s'étend de l'extrémité de tige proximale à la région de montage de ballonnet, la partie du fil s'étendant à travers la région de tige proximale est positionnée au sein de la lumière de gonflage, la partie du fil s'étendant à travers la région de tige distale est entrée en prise par adhérence à la région de tige distale,
la zone de montage de ballonnet ayant une longueur supérieure à la longueur de l'enveloppe de ballonnet à l'état brut de moulage, la longueur de la région de montage de ballonnet s'étendant d'une région de liaison proximale (54) située sur une extrémité distale de la région de tige proximale à une région de liaison distale (44) située sur une extrémité proximale de la région de tige distale ;
l'extrémité proximale de l'enveloppe de ballonnet est liée à la région de liaison proximale et l'extrémité distale de l'enveloppe de ballonnet est liée à la région de liaison distale,
l'enveloppe de ballonnet liée à la tige allongée a un état étendu de manière longitudinale, dans lequel l'enveloppe de ballonnet est configurée pour être étendue de manière longitudinale afin d'être montée sur la tige allongée, dans lequel la longueur de l'enveloppe de ballonnet correspond à la longueur de la région de montage de ballonnet,
l'intérieur de l'enveloppe de ballonnet est en communication fluidique avec la lumière de gonflage ; et
(iii) un moyeu proximal (15), l'extrémité de tige proximale est entrée en prise avec le moyeu et s'étend de manière proximale à partir de celui-ci, le moyeu définissant un orifice de gonflage (26), l'orifice de gonflage est en communication fluidique avec la lumière de gonflage,
lorsque le fil a une pointe proximale, la pointe proximale est entrée en prise fixement avec le moyeu.

2. Ensemble selon la revendication 1, dans lequel l'enveloppe de ballonnet liée à la tige allongée effectue une transition de manière gonflable depuis et entre un état de faible gonflage, un état de gonflage partiel, un état de gonflage complet, et un état de surgonflage,
la section d'épaulement distale conique définit un volume de section d'épaulement distale conique, la section d'épaulement proximale conique définit un volume de section d'épaulement proximale conique, dans l'état complètement gonflé le volume de section d'épaulement distale conique est inférieur au volume de section d'épaulement distale conique dans l'état partiellement gonflé, dans l'état complètement gonflé le volume de section d'épaulement proximale conique est supérieur au volume de section d'épaulement proximale conique dans l'état partiellement gonflé.

3. Ensemble selon la revendication 2, dans lequel, dans l'état de gonflage partiel, le méridien a un premier diamètre et est situé à une première distance de l'extrémité de tige distale, dans l'état complètement gonflé le méridien a un deuxième diamètre supérieur au premier diamètre et est situé à une deuxième distance de l'extrémité de tige distale, la deuxième distance est supérieure à la première distance, facultativement
dans lequel dans l'état surgonflé, le méridien conserve le même diamètre que dans l'état gonflé, le méridien est situé à une troisième distance de l'extrémité distale de l'enveloppe de ballonnet, la troisième distance est inférieure à la deuxième distance.

4. Ensemble selon la revendication 1, comprenant en outre :
un premier marqueur radio-opaque (90) et un deuxième marqueur radio-opaque (92), le premier marqueur radio-opaque est entré en prise avec le fil au niveau d'une position distale par rapport à la région de liaison proximale, et le deuxième marqueur radio-opaque est entré en prise avec le fil au niveau d'une position proximale par rapport à la région de liaison distale.

5. Ensemble selon la revendication 1, dans lequel la longueur de la tige allongée n'est pas supérieure à 75 cm.

6. Ensemble selon la revendication 5, dans lequel la région de tige distale a une longueur de région de tige distale non supérieure à 8 cm, la région de tige distale définissant une pointe en J atraumatique (16) au niveau de l'extrémité de tige distale, la pointe en J atraumatique ayant une longueur non supérieure à 5 cm.

7. Ensemble selon la revendication 6, comprenant en outre un redresseur de pointe en J, le redresseur de pointe en J comprenant une tige de décollement et une patte d'entrée en prise d'utilisateur fixée à la tige de décollement, la tige de décollement entrée en prise de manière coulissante avec et disposée de manière amovible autour d'une partie de la région de tige distale entre la pointe en J atraumatique et l'extrémité distale de l'enveloppe de ballonnet.

8. Ensemble selon la revendication 1, dans lequel le premier matériau et le deuxième matériau sont tous les deux des polyéthers blocs amide extrudés, le premier matériau ayant des propriétés de matériau différentes de celles du deuxième matériau.

9. Ensemble selon la revendication 8, dans lequel le premier matériau est le PEBAX^{®} et le deuxième matériau est le VESTAMIDE^{®} EVERGLIDE^{®} MED, et le matériau élastomère est l'uréthane.

10. Ensemble selon la revendication 1, dans lequel la longueur longitudinale de l'enveloppe de ballonnet à l'état brut de moulage est d'environ 70 mm et le diamètre du méridien est d'environ 8 mm, le col distal ayant une longueur d'environ 2 cm et le col proximal ayant une longueur d'environ 10 cm, dans l'état complètement gonflé le diamètre du méridien est compris entre environ 20 mm et environ 30 mm.

11. Ensemble selon la revendication 1, ayant un diamètre fonctionnel non supérieur à 4 Fr.

12. Ensemble selon la revendication 1, comprenant en outre des marqueurs visuels, les marqueurs visuels situés au niveau de zones spécifiques sur une surface externe de la région de tige proximale, chacun des marqueurs visuels indiquant la distance à laquelle l'enveloppe de ballonnet doit être avancée dans l'aorte afin d'être positionnée au niveau d'une zone anatomique correspondant à chacun des marqueurs visuels.

13. Ensemble selon la revendication 1, dans lequel la forme de cornet de glace de l'enveloppe de ballon est une forme auto-similaire.
